Europäisches Patentamt

European Patent Office

Office européen des brevets

(19)

(11) Publication number: **0 525 508 A2**

# EUROPEAN PATENT APPLICATION

(12)

(21) Application number: **92112033.3**

(22) Date of filing: **15.07.92**

(51) Int. Cl.⁵: **C12N 15/31**, A01N 63/04, C07K 7/10, C12N 1/21, C12N 5/10, A01H 5/00

(30) Priority: **19.07.91 GB 9115669**

(43) Date of publication of application:
**03.02.93 Bulletin 93/05**

(84) Designated Contracting States:
**AT BE CH DE DK ES FR GB GR IT LI NL PT SE**

(71) Applicant: **Max-Planck-Gesellschaft zur Förderung der Wissenschaften e.V. Bunsenstrasse 10 W-3400 Göttingen(DE)**

(72) Inventor: **Logemann, Susanne Dompfaffenweg 9 W-5000 Köln 30(DE)**
Inventor: **Schell, Josef Stefaan Carl-von-Linnéweg 12 W-5000 Köln 30(DE)**

(74) Representative: **Smolders, Walter et al c/o Sandoz Technology Ltd., Lichtstrasse 35 CH-4002 Basel(CH)**

(54) Anti-palnogenic protein of ustilago maydis and use thereof.

(57) Composition capable of controlling pathogens, methods of combatting plant pathogens and recombinant DNA sequences coding for amino acid sequences which display pathogenic activity against a wide range of fungi or bacteria, and which comprise amino acid sequences and amino acid sequences from position 1 through to 106 parts of SEQ ID No: 3 and modifications thereof, being the natural protein P4, secreted by Ustilago maydis.

EP 0 525 508 A2

The present invention relates to proteins which display activity against a broad range of pathogens. In particular, the invention relates to proteins capable of combatting plant pathogens.

## Background

It is known that certain strains of Ustilago maydis secrete proteins which are lethal to sensitive strains of U. maydis. Such proteins are encoded on specific segments of U. maydis double stranded RNA (dsRNA) viruses. Hitherto it was believed that these types of proteins were only toxic to certain smuts, i.e. fungi of the Ustilaginales. Thus it is known that certain strains of U. avenae, U. pullata, U. hordei, U. kolleri, U. nuda U. sphaerogena, U. striiformis, U. tritici and Sorosporium consanguineum are sensitive to the protein (P4) encoded by the U.maydis virus P4 (UmVP4).

It has now surprisingly been found that P4 protein, derivatives of it, and parts thereof have a useful activity against a broad range of pathogens including animal, human and plant pathogens, in particular fungal pathogens.

## Detailed Description

The present invention provides proteins including the amino acid sequence extending from amino acid position 15 to 96 of SEQ ID No. 1 and modifications thereof obtainable by adding, substituting and/or deleting one or more of the amino acids.

SEQ ID No. 1 describes a peptide of 96 amino acids in length, wherein the carboxy terminal amino acid sequence read "Glu Gly Ser". SEQ ID No. 1 was thought to be the correct peptide sequence for ustilin on the basis of manual sequence analysis of a cDNA clone which indicated that the ORF encoded a protein of length 96 amino acids (see SEQ ID No. 2 for the cDNA sequence). On checking the manual analysis with a machine assisted analysis of the ORF, it was found that the ORF encoded a protein of 106 amino acids in length (SEQ ID No. 3). This corrected sequence was verified subsequently; the discrepancy proved to be due to an error in reading during manual analysis wherein the C nucleotides at positions 278 and 279 of SEQ ID No. 2 were erroneously read as a double C. After machine analysis the nucleotides were found to form a triple C, as shown in positions 288, 289 and 290 of SEQ ID No. 4.

Such a discrepancy in the reading of the nucleotide sequence is however considered to have no bearing on the results of tests on the activity of the protein. It is believed that a protein having the sequence shown in SEQ ID No. 1 will exhibit activity similar to that of the natural protein having the sequence shown in SEQ ID No. 3

Thus, one preferred suitable modification of the peptide of SEQ ID No. 1 includes a protein having the amino acid sequence extending from position 15 to 93, preferably the amino acid sequence extending from position 1 to 93 of SEQ ID No. 1 and modifications thereof which may also be obtained through the addition, substitution and/or deletion of one or more amino acids.

A further modification of SEQ ID No. 1 includes the protein having an amino acid sequence extending from position 16 to 106 of SEQ ID No. 3, preferably the amino acid sequence extending from position 1 to 106 of SEQ ID NO. 3 and modifications thereof.

Amino acid sequences as mentioned above and modifications thereof (hereinafter proteins of the invention) can be prepared by way of standard techniques known in the art.

The proteins of the invention have a useful activity against pathogens including animal, human, and plant pathogens, in particular fungal pathogens.

Such modified sequences are conveniently functionally equivalent variants of the amino acid sequence extending from amino acid position 1 to 96 of SEQ ID No. 1, preferably the amino acid sequence extending from amino acid position 16 to 106 of SEQ ID No. 3 and more preferably the sequence extending from amino acid position 1 to 106 of SEQ ID No. 3. Included are functionally equivalent variants of the amino acid sequences extending from amino acid positions 1 or 15 to 93 of SEQ ID No. 1 and 1 or 16 to 106 of SEQ ID No. 3. The proteins of the invention preferably possess substantial sequence homology to the protein extending from amino acid position 15 to 93 of SEQ ID No. 1 - and/or to the protein extending from amino acid position 1 to 106 or to the protein extending from amino acid position 16 to 106 of SEQ ID No. 3. For the purposes of the description an homologous amino acid sequence is a sequence wherein one or more amino acids are added to, substituted into and/or deleted from a given sequence. According to a preferred embodiment of the invention the modified sequences are at least 70 % homologous to the proteins extending from amino acid position 15 to 96 of SEQ No. 1. According to another preferred embodiment of the invention, the modified sequences are at least 70 % homologous to the protein extending from position 15 to 93 of SEQ ID No. 1. According to a further preferred embodiment of the invention, the modified sequences are at least 70 % homologous to the protein extending from position 1 to 106 of SEQ ID No. 3. According to a further preferred embodiment of the invention, the modified sequences are at least 70 % homologous to the protein extending from position 16 to 106 of SEQ ID No. 3. Preferably the degree of homology of modified sequences with proteins of the invention is 80 % and most preferably is at least 90 %.

An embodiment of the invention as described above, relates to a protein comprising a protein according to the invention having at its N-terminal end linked thereto an oligopeptide having the amino acid sequence extending from amino acid position 1 to 14 of SEQ ID No. 1 or a modification of said oligopeptide in which one or more amino acids has been added, substituted, and/or deleted.

Modifications of the oligopeptide are conveniently functionally equivalent variants of the amino acid sequences extending from amino acid position 1 to 14 of SEQ ID No. 1 or from amino acid position 1 to 15 of SEQ ID No. 3.

The oligopeptides preferably possess substantial sequence homology to the oligopeptides extending from amino acid position 1 to 14 of SEQ ID No. 1 and/or the oligopeptides extending from amino acid position 1 to 15 of SEQ ID No. 3. Preferably, the modified sequences are at least 70 % homologous to the oligopeptides extending from amino acid position 1 to 14 of SEQ ID No. 1 or amino acid position 1 to 15 of SEQ ID No. 3, more preferably at least 80 % homologous, most preferably at least 90 %.

In a further embodiment of the invention, there is provided a method of combatting pathogens by applying to the pathogens or habitus thereof, a pathogen controlling amount of a protein according to the invention.

According to a preferred embodiment of the invention, the method comprises combatting plant pathogens, especially fungi, by applying to the plant pathogen or habitus thereof a plant pathogen controlling amount of a protein according to the invention.

Examples of plant pathogens that are susceptible to a protein of the invention, include in addition to the Ustilaginales fungi, fungi of the class of the Oomycetes particularly, Peronosporales such as Bremia spp, Pythium spp, Phythophtora spp, Peronospora spp; fungi of the class of the Deuteromycetes such as Rhizoctonia, Phoma spp, Fusarium spp, Botrytis spp, Verticillium spp, Didymella spp, Cryptocline spp and Alternaria spp; rust fungi, e.g. Uredinales fungi of the class of the Basidiomycetes, such as Puccinia spp; and bacterial plant pathogens, including bacteria of the family of the Pseudomonadaceae such as Xanthomonas spp.

Crops on which the method of the invention may be applied include dicotyledonous plants such as cabbage, carrots, tomato, pepper, cucumber, spinach, lettuce and tobacco, and monocotyledonous plants such as cereals, corn, leek and onion and the like.

The proteins of the invention may be employed in conventional manner. Against plant pathogens, the protein may for example, be applied in the form of a pesticidal composition comprising pathogen controlling proteins of the invention in a pathogen controlling amount in conjunction with agriculturally acceptable diluents. Such compositions may optionally comprise additional excipients such as stickers and the like. Such pesticide formulations may be obtained in a manner known per se; e.g. by employing an isolated protein of the invention as an active ingredient. Where a protein isolate is used, the protein is conveniently in essentially pure form.

The term "diluents" as used herein means a liquid or solid which is added to the protein to place it in a readily or more suitably applicable form such that the form does not adversely affect the active ingredient. Suitable diluents include water, xylene, talc, kaolin, diatomaceous earth, stabilising agents such as buffers, surfactants for enhancing contact between the proteins and the plant to be treated, UV protectants and the like.

Formulations used in spray form, such as water dispersible concentrates or wettable powders, may contain surfactants for wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol. Especially suitable surfactants include Tween-80 and Trixton-X-100.

The proteins according to the invention are typically stable through a wide pH range, e.g. the pH range of 4.0 to 9.0, and therefore water makes a suitable diluent. However, where appropriate the composition may additionally contain a buffer to maintain the composition within a desirable pH range. Any buffer solution may be used which is compatible with the proteins of the invention and which does not otherwise deleteriously affect the plant. Suitable buffers include, for example, a 50 mM tris/HCl, sodium succinate or sodium citrate and the like.

Typically, the amount of proteins according to the invention in the composition is between about 1 $\mu$q/ml and 500 $\mu$g/ml, lower amounts outside such a range are also acceptable, depending upon the particular protein being employed, the plant being treated, the conditions of treatment and the like.

Where the composition additionally contains a surfactant, the amount required is readily ascertainable by persons skilled in the art and will typically be in the range of from between about 0.001 and about 20 % by weight.

Further active ingredients, e.g., pesticides with similar or complementary activity or other beneficially acting materials such as insecticides, may be applied together with the proteins of the invention in order to increase their spectrum of activity or agricultural utility.

Proteins of the invention may also be employed in conjunction with other plant pathogen killing or plant pathogen controlling components in compositions suitable for use on dicotyledonous or monocotyledonous plants. Plant pathogen controlling agents may operate through a number of different ways such as through inhibiting growth in the plant pathogen, inhibition of key enzymes in biochemical pathways in plant pathogens and the like. Typically, plant pathogens against which the proteins of the invention are directed are fungi, and in particular, fungal species selected from the groups as outlined hereinbefore. The proteins of the invention may be applied to plants in compositions wherein one or more types of a plant pathogen controlling component, such as a killer protein may be present. Thus the composition to be applied may comprise, for example, as one component a P4 protein or a derivative thereof along with suitable additives, excipients and diluents as required. Alternatively, a mixture of two or more suitable "killer" proteins may be applied to plants in the form of a composition. A further alternative may comprise applying a first composition containing one killer protein and then applying a second composition containing a further killer protein immediately thereafter or after a suitable time interval. In such a manner, relays of killer proteins may be applied to plants. Examples of suitable additional killer proteins may include those designated as P1 and P6, also known as KP1 and KP6 respectively. Such additional killer proteins are known in the art, P6 for example, is described by Tao J. et al (Molecular and Cellular Biology [1990], pp. 1373-1381), pp. 1373-1381, incorporated herein by reference. A preferred composition is one which comprises a mixture of P4 and P6.

In a still further alternative the plant pathogen controlling or plant pathogen killing protein may be incorporated into a composition in an inactive state. The proteins may then be activated by adding a suitable further component which renders the protein into an active form. For instance, the inactive protein may be present in a prepro-protein or pro-protein form which, on the addition of suitable reagent, such as an appropriate enzyme, may be transformed into the active form. Suitable enzymes may include hydrolases such as site specific proteases and the like. The relative amount of each protein present in a composition is not critical. The proteins may be present in equal proportions relative to each other, however the relative proportion may differ depending on use, plant being treated, conditions of treatment and the like.

The concentration of proteins present in the composition may lie between 1 ug/ml and 500 ug/ml, however, concentrations lying outside this range may also be acceptable depending upon the particular mixture of killer proteins being employed, the plant being treated, conditions of treatment and the like.

Formulations as alluded to above containing proteins of the invention, a diluent and other additives are then applied to the plant, seeds or plant loci. Application of the composition may be carried out in accordance with accepted practice well known to those skilled in the art such as by spraying onto plants. It will be appreciated that the amount of compositions to be applied will depend on what is being treated (i.e. plant, seed or soil), the type of treatment (e.g. spraying, dusting, seed dressing), purpose of the treatment (prophylactic, therapeutic), type of pathogen to be treated and the application time.

The pesticidal compositions of this invention are desirably applied to plants that are attacked by pathogens at a rate ranging from 100 to 5000 1/ha. It may be desired with many plants to repeat the treatment after several weeks if necessary to provide the desired protection from the pathogens present on the plant. Moreover, as a preventive measure, plants that are susceptible to pathogen attack may be sprayed or otherwise treated with the pesticidal compositions of this invention prior to the first sign of the presence of the pathogen or of symptoms of infection of the plant by the pathogen.

A particular method for use of the proteins of the invention in the control of plant pathogens is applying the proteins of the invention in the form of agriculturally acceptable cell systems that have been transformed or transfected to produce a protein of the invention. Examples of suitable host cells comprise yeasts, bacteria including Bacillus spp such as Bacillus thuringiensis, B. cereus, B. subtilis and Pseudomonas spp such as Pseudomonas fluorescens, E. coli and plant cells.

Such host cells once transformed or transfected, can be conveniently grown utilising fermentation techniques in conventional manner in an appropriate nutrient containing medium.

A suitable suspension concentrate of such cells can be obtained by evaporation of fermentation liquor to a desired concentration. Additives may be added where desired.

In an analogous fashion, a wettable powder formulation may be obtained by spray drying the fermentation liquor, which may additionally comprise such components as emulsifiers, UV protectants and the like, and pulverisation of the solid obtained. Thus obtained formulations contain a substantial proportion of the components of the nutrient medium as diluent.

Alternatively, the fermentation liquor may be centrifuged to separate out the bigger particles of the nutrient medium and then be converted, as mentioned above, to a suitable suspension concentrate or wettable powder formulation. Suitable formulations comprise in general 1 x 10 to 1 x 10 CFU's per mg or ml formulated product.

The proteins of the invention may be produced through recombinant DNA techniques.

In a further embodiment of the invention there are provided recombinant DNA molecules comprising DNA sequences coding for proteins of the invention. An example of a DNA molecule coding for the amino acid sequence extending from amino acid position 15 to 96 of SEQ ID No. 1 is shown in DNA SEQ ID No. 2. SEQ ID No. 4 describes a DNA molecule coding for the amino acid sequence extending from amino acid position 16 to 106 of SEQ ID No. 3 as well as the sequence extending from amino acid position 1 to 106 of SEQ ID No. 3. In view of the redundancy of the genetic code, most triplets of SEQ ID No. 2 and 4 coding for a particular amino acid can be exchanged for other triplets coding for that same particular amino acid, without effect on the sequences of the proteins the DNA molecule codes for. Thus, it can be seen, for example, that the first fourteen triplets of SEQ ID No. 4 are different from the first fourteen triplets of SEQ ID No. 2, yet the difference in nucleotide triplets does not make a difference to the order of the actual sequence of amino acids obtained in a protein of from 1 to 96 amino acids, or form 1 to 93 amino acids in length as illustrated by SEQ ID No. 1.

The DNA sequences coding for a protein according to the invention are hereinafter designated recombinant DNA sequences according to the invention. The recombinant DNA sequences according to the invention can be incorporated into a vector through conventional recombinant techniques and used to transform or transfect host cells, e.g. micro-organisms such as E. coli or B. subtilis in order to produce the proteins according to the invention in useful quantities. The term vector employed herein refers to a vehicle by means of which DNA fragments can be incorporated into host organisms. The recombinant DNA according to the invention can also be incorporated into the genome of a plant in order to give the plant reduced susceptibility to plant pathogens.

The invention therefore also provides recombinant DNA comprising a recombinant DNA sequence according to the invention operatively linked to a regulatory sequence (i.e. promoter) capable of promoting its transcription in a host cell and, where desired, a terminator sequence.

The term "promoter" as used herein refers to the nucleotide sequence upstream from the transcriptional start site and containing all the regulatory signals required for expression, including the region coding for the leader sequence of mRNA, which leader sequence comprises the ribosomal binding sites and initiates translation at the AUG start codon. Examples of promoters are known in the art.

The cloning techniques themselves are known or adaptable from known techniques. DNA comprising sequences coding for proteins according to the invention are incorporated under the control of appropriate regulatory sequences into plasmids to form vectors which are transformed or transfected into cells to thereby enable the transformed cells to produce the proteins. The DNA may optionally contain alternate signal sequences for targeting the protein expression product to different cell compartments or to an extracellular location. Typically the DNA comprises a recombinant DNA molecule comprising a first DNA sequence promoting the transcription of a second linked DNA sequence, wherein this second DNA sequence comprising a DNA sequence coding for a protein according to the invention. Preferably, the recombinant DNA molecule encodes an amino acid sequence having substantial sequence homology to proteins of the present invention as hereinbefore described. Conveniently they will have 70 % homology to such proteins, preferably at least 80 % homology, particularly at least 90 % homology. Typical examples of such recombinant DNA molecules include those encoding a protein containing the amino acid sequence of a protein according to the invention wherein the N-terminal amino acid is linked to an oligopeptide having the amino sequence extending from amino acid position 1 to 14 of SEQ ID No. 1 or an amino acid sequence extending from position 1 to 15 of SEQ ID No. 3 or modifications thereof as mentioned hereinbefore.

Production of the proteins according to the invention involves transforming or transfecting a host with a vector comprising a recombinant DNA sequence according to the invention and subsequently culturing the transformed or transfected host cell to produce the protein. While a variety of transformed or transfected cell systems may be employed, it is generally preferred to transform or transfect bacterial cells of either the gram-negative or gram-positive type. One preferred type of gram-negative bacteria is E. coli with which considerable experience in biotechnology has already been achieved and for which a wide variety of suitable and operatively functional plasmid and transfer expression vector systems are known and available.

Pseudomonas fluorescens represents another type of gram-negative bacteria into which plasmids carrying the protein sequence have been incorporated. Further suitable host cells include Bacillus thuringiensis (B.t.), B. cereus, and B. subtilis. Typical promoters suitable for use in transformation of E. Coli include $P_{tac}$, $P_{trp}$, $P_{trc}$ and $P_1$. All of which are available from Pharmacia. The proteins according to the invention can be isolated from the cells or from the culture medium of thus transformed or transfected host cells in a manner known per se and can be purified in conventional manner.

The DNA sequences according to the present invention can also be inserted into the genome of a plant that is vulnerable to pathogens. Any suitable method may be employed for such incorporation of the DNA encoding a protein according to the invention into a host plant cell genome, such as for example, via Ti plasmid of Agrobacterium tumefaciens, electroporation, electrotransformation, ballistic gun, micro-injection, viral invention or the use of chemicals that induce or increase free DNA uptake, and the like. Such procedures and the use of such in the transformation of plants are well known to one skilled in the art. Preferably, the DNA sequence encoding the proteins according to the invention, will be operatively associated with appropriate regulatory sequences, such as for example, 5' promoter and 3' regulatory (terminator) sequences and signal sequences for intracellular targeting or extracellular targeting which are functional in plants, and the whole will be incorporated into a vector. The promoter may express the DNA constitutively or differentially. Examples of promoters differentially regulating DNA expression are promoters inducible by disease vectors, e.g. so-called wound or pathogen inducible promoters. Examples of constitutive promoters include viral promoters such as 35S-CaMV or modifications thereof. Such transformation of plant cells, followed by regeneration and development of cells into whole plants, enables the DNA sequence to become a stable and permanent part of the plant genome, such that it is passed on from one generation to the next via mitosis and meiosis and upon expression results in a protein endowing the plant with inheritable reduced susceptibility to plant pathogen attack.

The invention accordingly also provides transgenic organisms comprising recombinant DNA according to the invention.

The following examples illustrate the invention.

## Example 1: P 4 purification

The UmVP4 is grown in minimal medium (salt medium and sugar). The P4 protein is produced by UmVP4 as a preprotoxin and secreted as a mature protein to the fungal growth medium. P4 purification is achieved by concentrating the proteins of 4 l of the fungal medium by 2 ultrafiltrations to a volume of 100-200 ml, followed by an anion exchange step, a kation exchange step and a size fractionation.

For the anion exchange step the concentrate is adjusted to pH 8 employing 50 mM Tris and stirred with 10 g DE52-cellulose for 3-4 hours. The suspension is filtered to remove the powders, subjected to ultrafiltration, dialysed for 12 hours to 50 mM sodium acetate at pH 4 and then pumped over a column containing S-Sepharose fast flow. The column is washed with 50 mM sodium acetate at pH4 and then eluted with a linear gradient of 0.2 to 0.6 M NaCl in 50 mM NaCl (2 x 55 ml). The active fractions are concentrated by lyophilisation and dialysed to water. The dialysate is size fractioned on a highload column with Superdex 75 in 100 mM Tris at pH 8 and 150 mM NaCl. The thus obtained active fraction containing purified P4 has the follow purification date:

| | |
|---|---|
| starting volume: | 4 l |
| input K-Units* | 7700 U |
| = total protein | 5 mg |
| output K-Units* | 400-500 U |
| = protein amount | 50 $\mu$g |
| $\mu$g P4/K-Unit* | 0.1 $\mu$g |
| K-Units/$\mu$g P4 | 10 U |

* the unit required to produce an inhibitory zone of diameter in the bioassay according to Levine et al (Nucleic Acids Research 6 3717-3731 (1979).

This fraction can be used to produce polyclonal antisera in rabbits.

## Example 2: Isolation of dsRNA M2-segment

The M2-segment of UmVP4 encodes the P4 gene as dsRNA. It is isolated by destroying the fungal cells and concentration of viral particles by NaCl/PEG precipitation of a cellfree extract. After proteinase K digestion and phenol and chloroform extractions, the viral genome is ethanol precipitated and then size fractioned on a PAA gel, stained with EtBr and the M2-segment is excised. After elution from the acrylamide and ion exchange chromatographic purification the ds-RNA M2 segment is ready for cDNA synthesis.

### Example 3 - cDNA

The first cDNA clones are synthesised using random primer.

Approx. 15 ug of random primer are annealed to 1 - 2 ug of M2-segment of UmVP4, reverse transcribed and the second strand synthesised using RNAse H/E.coli DNA polymerase technique. Thus obtained short cDNA clones of about 200 bp in size are cloned in E. coli. Clones hybridising to UmVP4 genome in Northern blot experiments are sequenced. The sequence analysis was used to design oligonucleotides which represent a short sequence either on the coding strand or the complementary strand.

These synthetic oligonucleotides are annealed to the M2-segment as specific primers for a new set of cDNAs after chemical denaturation of the dsRNA by heating 2 $\mu$g M2-segment, 10 $\mu$l water, 4 $\mu$g oligonucleotides and 100 $\mu$l DMSO for 30 minutes at 65°C, followed by precipitation, centrifugation and drying of the pellet.

4 $\mu$l 5 x reaction buffer suitable to the enzyme (250 mmol Tris (pH8), 200 mmol KCl and 30 mmol MgCl$_2$), 2 $\mu$l 0.1 M DTT, 1 $\mu$l 10 mM nucleotides, 0.5 $\mu$l Gene 32 protein, 10 $\mu$l water and 1 $\mu$l MMLV Superscript (reverse transcriptase) are then mixed and preheated to 38°C.

The preheated mixture is added to the pellet (nucleic acid dissolved therein) and the mixture incubated at 38° for 5 minutes. The temperature is then increased every 3 minutes by 1°C until a temperature of 44°C is reached.

0.5 $\mu$l MMLV are then added and the mixture kept for 30 minutes at 44°C. The thus obtained cDNA first strand is amplified by PCR technique applying further oligos. Sequence analysis of the first and the second set of cDNA clones leads to the sequence data for the mature P4 protein. From these cDNA clones the P4 gene is constructed and ligated to different regulatory sequences suitable for expression in plants and microorganisms.

### Example 4 - P4-Sequence

The P4 protein is purified and used to produce polyclonal antibodies. These antibodies are used to prove the specificity of the obtained sequence data by two methods.

1. The putative P4 sequences are ligated to bacterial regulatory sequences resulting in an expression vector. By Western Blot technique it is shown that the expressed bacterial products cross-react with antibodies against the P4. In cases in which the P4 sequence or an active part thereof is expressed, the bacterial product will exhibit P4 activity.

2. The cDNA sequences according to Example 3 are ligated to vectors which allow "in vitro" transcription and the "in-vitro" RNA is translated in a reticulocyte lysate. These "in vitro" translation products as well as "in vitro" translation products from the M2-segments cross-react with the antibodies.

3. The P4 sequence can also be determined by protein sequencing of the purified P4 and comparison of the data obtained with the previously obtained cDNA sequence data.

### Example 5-E. coli transformation

The cDNA sequence 43 to 288 from DNA SEQ ID No. 2 is cloned into vector pUC19 (at the complementary strand) and then via a BamH1 restriction fragment is cloned into vector pEV-vrf to give pEV-2-18 in conventional manner. (Versatile Expression Vector for high level synthesis of clone gene products by R. Crowl et al in GENE 38 (1985) pp. 31-38). The thus transformed E. coli comprising the cDNA sequence of DNA SEQ ID No. 5 is grown in an appropriate medium, centrifuged, ultrasonicated and the cell lysate subjected to an in vitro test for Ustilago maydis on agar medium. The cell lysate comprising the protein of SEQ ID No. 6 has fungicidal activity.

### Example 6-E. coli transformation

The cDNA sequence 53 to 282 from DNA SEQ ID No. 4 are cloned into plasmid pUC19 to give pUC2-17 (in the complementary strand). PCR clone, pUC83G, is generated from PCR experiments and includes DNA sequence 175-330 from DNA SEQ ID No. 4. cDNA sequences 53-282 from DNA SEQ ID No. 4 are cloned into pEVvrf2 via a BamH1 restriction fragment to give pEV2-17. Sequences corresponding to that of 175-282 of SEQ ID No. 4 are removed from pEV2-17 and substituted with DNA sequence 175-313, which is excised from pUC83G via a Snab1 restriction fragment thereby providing pEV2-19. The cell lysate of primary transformants including pEV2-19 has fungicidal activity.

### Example 7-E. coli transformation

The DNA sequence for amino acids 2-15 of SEQ ID No. 3 and the DNA sequence 1-49 of SEQ ID No. 4 are deduced from N-terminal protein sequencing data and then added to DNA sequences 53-313 of SEQ ID No. 4 in pUC2-19. Simultaneously, DNA sequences 284-306 (excluding No. 289) along with eight additional base pairs are added to the C terminus using standard PCR techniques. The resulting PCR product includes DNA sequences 1-306 of SEQ ID No. 4 and codes for amino acids 1-94 and three additional amino acids. This is cloned into vector pET-11t, a derivative of pET 11 d available from Novagen, resulting in pET2-31. The cell lysate of transformants including pET2-31 has fungicidal activity.

### Example 8-E. coli transformation

Clone pUCQ is constructed from pUC83G using standard PCR techniques. pUCQ includes the DNA sequence 175-337 of SEQ ID No. 4. Sequences from clone pUCQ are inserted into pUC2-30 and replace sequences 175-306 of pUC2-30. This results in pUC2-40 which includes sequences 1-337 of SEQ ID No. 4. Sequences 8-337 are excised from pUC2-40 using an Sph1 restriction site and inserted into pET 11t, giving rise to pET2-40. pET2-40 codes for amino acids 1-106 of SEQ ID No. 4.

### Example 9: Subcloning of the Ustilin Gene and Transformation into Plants.

cDNA clone pUC2-40X is subjected to standard PCR reaction conditions. Primers are tailored so as to be homologous to the start and stop codon.

Primer ust-1:

**d(GCAGATATCATGCTTGGAATTAATTGCAGAGGG) - SEQ ID NQ: 7**
This creates an EcoRV site just proximal to the start codon and in the process mutates the BamHI site as in SEQ ID No: 4 to a non-cutable site.

Primer Ust-2:

**d(GCAACCTGCAGCTCAACACGAGTTTACG) - SEQ ID NQ: 8**
This creates a PstI site just distal to the stop codon. Such primers are used in standard PCR experiments in which pUC2-40X can be used as a template. The resulting PCR fragment is isolated from the gel using a DEAE membrane, NA-45, (available from the company Schleicher and Schüll) and is cloned into a SmaI linearized pBluescript to generate plasmid pUST-2. Added restriction sites, EcoRV and PstI, facilitate the construction of further plasmids. The ustilin gene is excised from plasmid pUST-2 via EcoRV-PstI digestion. The fragment is isolated and inserted into SmaI-PstI linearised pZU-B following the method outlined by Gielen JJL et al (Bio/Technology 9: 1363-1367 [1991]) resulting in recombinant plasmid pUST-2B. The chimeric cassette containing the 35S-Ω promoter, the ustilin gene and the NOS terminator is excised from plasmid pUST-2B via a BamHI/XbaI digestion. The isolated chimeric gene cassette is inserted into the BamHI/XbaI linearized pBIN19 to create binary transformation vector pUST-2BB. Resulting plasmid pUST-2BB is used in plant transformation experiments following the methods as outlined by Horsch et al. (Plant Molecular Biology Manual AS: 1-9 (1988), Kluwes Academic Publishers, Dordrecht, Netherlands).

### Plant Pathogen Controlling Activity

Culture medium from U. maydis harbouring the toxin producing UmVP4 strain is lyophilized and tested against the following pathogens on agar culture medium:
Phoma lingam
Alternaria brassicicola

Pythium violae
Phytophthora porri
Xanthomonas carotae
Xanthomonas vesicatoria
Cryptocline cyclaminis
Alternaria dauci
Alternaria pluriseptata
Didymella lycopersici
Fusarium oxysporum conglutinans fysio 1
Botrytis aclada
Pythium ultimum
Verticillium lycopersici
Rhizoctonia solani
Puccinia porri
Peronospora farinosa f.sp. spinaceae
Xanthomonas maltophilia

Analogous tests are run employing sterile growth medium and culture medium from U. maydis not harbouring the UmVP4 strain as standards.

The tested media controls the growth of the pathogens whereas the standards do not have an effect on the growth on the pathogens.

The test against the fungal pathogens is repeated employing an aqueous solution of the purified P4 protein.The fungicidal activity of the tested protein is confirmed.

## Pesticidal Compositions

### Example A

| | |
|---|---|
| P4 protein | 50 µg/ml water |

### Example B

| | |
|---|---|
| P4 protein | 50 µg/ml water |
| Buffer | 10 mM sodium phosphate pH 7.0 |

### Example C

| | |
|---|---|
| P4 protein | 50 µg/ml water |
| Triton X-100 | 0.05 % wt/vol |

### Example D

| | |
|---|---|
| P4 protein | 50 µg/ml water |
| Buffer | 10 mM sodium phosphate pH 7.0 |
| Triton X-100 | 0.05 % wt/vol |

**SEQUENCE LISTING**

SEQ. ID NO: 1
Sequence type: protein
Sequence length: 96 amino acids
Original Source Organism: Ustilago maydis virus P4

```
 1  Leu Gly Ile Asn Cys Arg Gly Ser Ser Gln Cys Gly Leu Ser Gly Gly  16

17  Asn Leu Met Val Arg Ile Arg Asp Gln Ala Cys Gly Asn Gln Gly Gln  32

33  Thr Trp Cys Pro Gly Glu Arg Arg Ala Lys Val Cys Gly Thr Gly Asn  48

49  Ser Ile Ser Ala Tyr Val Gln Ser Thr Asn Asn Cys Ile Ser Gly Thr  64

65  Glu Ala Cys Arg His Leu Thr Asn Leu Val Asn His Gly Cys Arg Val  80

81  Cys Gly Ser Asp Pro Leu Tyr Ala Gly Asn Asp Val Ser Glu Gly Ser  96
```

SEQ ID NO: 2
Sequence type: nucleotide
Sequence length: 288 nucleotides
Strandedness: single
Molecule type: cDNA
Original Source Organism: Ustilago maydis virus P4

```
  1  CTC GGC ATA AAC TGT CGA GGC TCT AGC CAG TGT GGC CTC AGC   42
 43  GGC GGG AAC CTT ATG GTC CGA ATA AGA GAT CAG GCA TGT GGT   84
 85  AAC CAA GGC CAA ACA TGG TGT CCT GGT GAA CGG CGT GCT AAG  126
127  GTC TGT GGG ACT GGC AAT AGC ATC TCT GCT TAT GTT CAG TCT  168
167  ACC AAC AAT TGT ATA TCA GGG ACA GAG GCC TGT CGC CAT TTG  210
211  ACT AAC CTC GTT AAC CAT GGT TGT AGA GTC TGT GGC AGC GAC  252
253  CCA CTC TAT GCC GGG AAT GAT GTG TCC GAG GGC AGT          288
```

**SEQ ID NO:  3**

Sequence type:  protein Sequence length:  106 amino acids

Original Source Organism:  <u>Ustilago maydis</u> virus P4

```
1   MET LEU GLY ILE ASN CYS ARG GLY SER SER GLN CYS GLY LEU SER GLY  16
17  GLY ASN LEU MET VAL ARG ILE ARG ASP GLN ALA CYS GLY ASN GLN GLY  32
33  GLN THR TRP CYS PRO GLY GLU ARG ARG ALA LYS VAL CYS GLY THR GLY  48
49  ASN SER ILE SER ALA TYR VAL GLN SER THR ASN ASN CYS ILE SER GLY  64
65  THR GLU ALA CYS ARG HIS LEU THR ASN LEU VAL ASN HIS GLY CYS ARG  80
81  VAL CYS GLY SER ASP PRO LEU TYR ALA GLY ASN ASP VAL SER ARG GLY  96
97  GLN LEU THR VAL ASN TYR VAL ASN SER CYS *                        106
```

SEQ ID NO:  4 Sequence type:  nucleotide Sequence length:  337 nucleotides Strandedness:  single Molecule type:  cDNA Original Source Organism:  Ustilago maydis virus P4

```
  1  CTCGAGC ATG CTT GGA ATT AAT TGC AGA GGA TCC TCA CAA TGC    43
 44  GGA CTT TCT GGC GGG AAC CTT ATG GTC CGA ATA AGA GAT CAG    85
 86  GCA TGT GGT AAC CAA GGC CAA ACA TGG TGT CCT GGT GAA CGG   127
128  CGT GCT AAG GTC TGT GGG ACT GGC AAT AGC ATC TCT GCT TAT   169
170  GTT CAG TCT ACC AAC AAT TGT ATA TCA GGG ACA GAG GCC TGC   211
212  CGC CAT TTG ACT AAC CTC GTT AAC CAT GGT TGT AGA GTC TGT   253
254  GGC AGC GAC CCA CTC TAT GCC GGG AAT GAT GTG TCC CGA GGG   295
296  CAG TTG ACT GTC AAC TAC GTA AAC TCG TGT  TGATAAGAAT TC    337
```

SEQ ID NO: 5 Sequence type:    nucleotide
Sequence length:  267 nucleotides
Original source organism:  <u>ustilago maydis</u> virus PE

```
  1  ATG AAT AGA ATT CGG ATC CCC GGC GGG AAC CTT ATG GTC CGA   42
 43  ATA AGA GAT CAG GCA TGT GGT AAC CAA GGC CAA ACA TGG TGT   84
 85  CCT GGT GAA CGG CGT GCT AAG GTC TGT GGG ACT GGC AAT AGC  126
127  ATC TCT GCT TAT GTT CAG TCT ACC AAC AAT TGT ATA TCA GGG  168
167  ACA GAG GCC TGT CGC CAT TTG ACT AAC CTC GTT AAC CAT GGT  210
211  TGT AGA GTC TGT GGC AGC GAC CCA CTC TAT GCC GGG AAT GAT  252
253  GTG TCC GAG GGC AGT                                       267
```

SEQ ID NO: 6 Sequence type:    protein
Sequence length: 89 amino acids
Original Source Organism:  <u>Ustilago maydis</u> virus P4

```
 1  Met Asn Arg Ile Arg Ile Pro Gly Gly Asn Leu Met Val Arg Ile Arg  16

17  Asp Gln Ala Cys Gly Asn Gln Gly Gln Thr Trp Cys Pro Gly Glu Arg  32

33  Arg Ala Lys Val Cys Gly Thr Gly Asn Ser Ile Ser Ala Tyr Val Gln  48

49  Ser Thr Asn Asn Cys Ile Ser Gly Thr Glu Ala Cys Arg His Leu Thr  64

65  Asn Leu Val Asn His Gly Cys Arg Val Cys Gly Ser Asp Pro Leu Tyr  80

81  Ala Gly Asn Asp Val Ser Glu Gly Ser                              89
```

13

SEQ ID NO:  7

Sequence type:     nucleotide Sequence length:   33 nucleotides

Source:            synthetic DNA

1   GCAGATATC ATG CTT GGA ATT AAT TGC AGA GGG     33

SEQ ID NO:  8

Sequence type:     nucleotide Sequence length:   28 nucleotides

Source:            synthetic DNA

1   GCAACCTGCAGCTCAACACGAGTTTACG     28

## Claims

1.  A method of combatting plant pathogens which comprises applying to the plant pathogen or habitus thereof a protein capable of controlling plant pathogens wherein the amino acid sequence thereof includes the amino acid sequence of from position 15 up to and including amino acid position 96 of SEQ ID No. 1 or a functionally equivalent modification thereof in which one or more amino acids have been added, substituted, and/or deleted.

2.  A method according to Claim 1, wherein the amino acid sequence of the protein extends from amino acid position 15 to amino acid position 93 of SEQ ID No. 1.

3.  A method according to Claim 1, wherein the amino acid sequence of the protein extends from amino acid position 15 to amino acid position 106 of SEQ ID No. 3.

4.  A method according to any one of the preceding claims, wherein the amino acid sequence of the protein has at least 70 % homology to the amino acid sequences extending from amino acid position 15 to 96 of SEQ ID No. 1 or from amino acid position No. 15 to 106 of SEQ ID No. 3.

5.  A method according to Claim 4 wherein the amino acid sequence of the protein has at least 80 % homology to the amino acid sequences extending from amino acid position 15 to 96 of SEQ ID No. 1 or from amino acid position 15 to 106 of SEQ ID No. 3

6.  A method according to Claim 5 wherein the amino acid sequence of the protein has at least 90 % homology to the amino acid sequences extending from amino acid position 15 to 96 of SEQ ID No. 1 or from amino acid sequence position 15 to 106 of SEQ ID No. 3.

7.  A method according to any one of Claims 1 to 6, which comprises applying a protein comprising an amino acid sequence as defined in any one of the preceding claims wherein an oligopeptide having the amino acid sequence extending from amino acid position 1 to amino acid position 14 of SEQ ID No. 1 or an amino acid sequence extending from position 1 to 15 of SEQ ID No. 3 or a modification of said oligopeptide in which one or more amino acids have been added, substituted and/or deleted, is linked to the N-terminal end of the said protein.

14

8. A method according to any one of Claims 1 to 7 wherein the plant pathogen is a fungus selected from the class of the Oomycetes, Deuteromycetes or Basidiomycetes; or belongs to the Uredinales or is a bacterium belonging to the Pseudomonadaceae.

9. A pathogenically effective composition comprising a pathogen controlling amount of a protein as stated in Claims 1 to 7.

10. The composition of Claim 9 comprising additionally the protein KP 6 or modifications thereof wherein one or more amino acid sequences have been added, substituted, and/or deleted.

11. Recombinant DNA comprising a DNA sequence coding for an amino acid sequence as stated in Claims 1 to 7.

12. Recombinant DNA according to Claims 11, further comprising a DNA sequence capable of promoting the transcription of the recombinant DNA and to which the further DNA sequence is operably linked.

13. A recombinant DNA according to Claim 12, wherein said DNA sequence is under the control of DNA operably capable of causing expression of said DNA sequence in E. coli.

14. Recombinant DNA according to Claim 12, wherein said DNA sequence is under the control of DNA operably capable of causing expression of said DNA sequence in a plant vulnerable to a pathogen.

15. Recombinant DNA according to Claim 14, wherein said DNA sequence further comprises an alternate signal sequence capable of targeting the protein to an extracellular location.

16. A process for the production of a protein stated in Claims 1 to 7, which comprises transforming or transfecting a cell with a vector containing recombinant DNA according to any one of Claims 11 to 15 and culturing resulting cells to produce said protein.

17. Micro-organisms, plant cells, and plants transformed with a vector containing recombinant DNA according to any one of Claims 11 to 15 and progeny thereof.

18. A method of producing a transgenic plant cell or plant having the potential to regenerate into a plant demonstrating resistance or tolerance to plant pathogens comprising transforming the plant cell or plant with a vector containing recombinant DNA according to either Claim 14 or Claim 15.

19. A protein having the amino acid sequence extending from position:
    i) 15 to 96 of SEQ ID No. 1;
    ii) 1 to 96 of SEQ ID No. 1;
    iii) 15 to 106 of SEQ ID No. 3;
    iv) modifications of i), ii) or iii), wherein one or more amino acids have been added, substituted and/or deleted.

20. The protein having the amino sequence extending from position 1 to 106 of SEQ ID No. 3 in substantially pure form.

21. A protein having substantial sequence homology to the protein according to Claim 20.